# NEW EUROPEAN PATENT SPECIFICATION

(11) **EP 3 433 616 B2**
(45) Date of publication and mention of the opposition decision: **24.07.2024**
(45) Mention of the grant of the patent: 30.12.2020
(21) Application number: 17712782.6
(22) Date of filing: 22.03.2017
(51) Int. Cl.: G01N 33/574, G01N 33/68

(54) **USE OF NUCLEOSOME-TRANSCRIPTION FACTOR COMPLEXES FOR CANCER DETECTION**
VERWENDUNG VON NUKLEOSOM-TRANSCRIPTIONSFAKTOR COMPLEXE ZUR KREBSERKENNUNG
UTILISATION DE COMPLEXES DE FACTEUR DE TRANSCRIPTION-NUCLÉOSOME POUR LA DÉTECTION DU CANCER

(30) Priority: 22.03.2016 GB 201604806
(43) Date of publication of application: 30.01.2019
(60) Divisional application: 20210821.3
(73) Proprietor: Belgian Volition SPRL, 5032 Isnes (BE)
(72) Inventor: MICALLEF, Jacob Vincent, 5032 Isnes (BE); REIS-FILHO, Jorge, 5032 Isnes (BE)
(74) Representative: Sagittarius IP
(86) International application number: PCT/EP2017/056851
(87) International publication number: WO 2017/162755

(56) References cited:
- WO-A2-2013/084002
- PATRICIA L KANDALAFT, GOWN ALLEN M: "CAP Laboratory Improvement Programs Practical Applications in Immunohistochemistry Carcinomas of Unknown Primary Site", ARCH PATHOL LAB MED, vol. 140, no. 6, 1 June 2016 (2016-06-01), pages 508 - 523
- S.K Bhatia et al (1987) Cancer 59(6) 1170-1172
- BALLARÉ CECILIA ET AL: "Nucleosome-Driven Transcription Factor Binding and Gene Regulation", MOLECULAR CELL, CELL PRESS, CAMBRIDGE, MA, US, vol. 49, no. 1, 21 November 2012 (2012-11-21), pages 67 - 79, XP028963344, ISSN: 1097-2765, DOI: 10.1016/J.MOLCEL.2012.10.019
- MATTHEW W. SNYDER ET AL: "Cell-free DNA Comprises an In Vivo Nucleosome Footprint that Informs Its Tissues-Of-Origin", CELL, vol. 164, no. 1-2, 14 January 2016 (2016-01-14), US, pages 57 - 68, XP055367434, ISSN: 0092-8674, DOI: 10.1016/j.cell.2015.11.050
- CHRISTIN WITTWER ET AL: "Circulating nucleosomes and immunogenic cell death markers HMGB1, sRAGE and DNAse in patients with advanced pancreatic cancer undergoing chemotherapy", INTERNATIONAL JOURNAL OF CANCER, vol. 133, no. 11, 9 August 2013 (2013-08-09), US, pages 2619 - 2630, XP055367098, ISSN: 0020-7136, DOI: 10.1002/ijc.28294
- MOTOKI TAKAKU ET AL: "GATA3-dependent cellular reprogramming requires activation-domain dependent recruitment of a chromatin remodeler", GENOME BIOLOGY, vol. 17, no. 1, 27 February 2016 (2016-02-27), XP055366461, DOI: 10.1186/s13059-016-0897-0
- IVANA V. YANG ET AL: "Epigenetic Control of Gene Expression in the Lung", AMERICAN JOURNAL OF RESPIRATORY AND CRITICAL CARE MEDICINE., vol. 183, no. 10, 15 May 2011 (2011-05-15), US, pages 1295 - 1301, XP055367350, ISSN: 1073-449X, DOI: 10.1164/rccm.201010-1579PP
- ZE-WEI LIN ET AL: "The Expression Levels of Transcription Factors T-bet, GATA-3, ROR[gamma]t and FOXP3 in Peripheral Blood Lymphocyte (PBL) of Patients with Liver Cancer and their Significance", INTERNATIONAL JOURNAL OF MEDICAL SCIENCES, vol. 12, no. 1, 1 January 2015 (2015-01-01), AU, pages 7 - 16, XP055367446, ISSN: 1449-1907, DOI: 10.7150/ijms.8352

## Description

### FIELD OF THE INVENTION

The invention relates to a method for detecting cancer, in particular identifying the primary cancer in a subject with metastatic cancer disease of unknown primary, by means of a tissue specific transcription factor-nucleosome adduct or transcription cofactor-nucleosome adduct as a biomarker in a biological fluid.

### BACKGROUND OF THE INVENTION

A wide range of immunoassay blood tests are in common clinical use to address a wide range of clinical needs. A few illustrative examples include use to detect or investigate viral disease, myocardial infarction, thyroid disease, infertility, gynaecological conditions, inflammatory response, immune status, allergies, drug status (pharmaceutical, androgenic, performance enhancing and recreational) and many more. Automated immunoassays systems offered by large diagnostics companies have menus of hundreds of immunoassay tests. The many advantages of immunoassay tests include their extreme analytical sensitivity and specificity combined with robustness and reliability; together with a minimally invasive patient format that requires only a drop of blood, extreme flexibility allowing testing to be performed in large laboratories or in the doctor's office or in a home use format and all at low cost. Despite this, only one immunoassay blood test is in common clinical use for cancer detection; the Prostate Specific Antigen (PSA) test for prostate cancer. Other classical protein cancer biomarkers, such as CA125, CA19.9, CEA (carcinoembryonic antigen) and AFP (alpha-fetoprotein), are used to monitor cancer treatment but are not recommended for cancer detection due to their poor clinical accuracy.

Non-immunoassay blood based cancer detection methods based on circulating tumor cell detection are in development but not in common clinical use. Similarly, methods for circulating tumour DNA (ctDNA), RNA or other circulating nucleic acids are in development but these are also not in widespread clinical use. There are a wide variety of circulating nucleic acid methods. Some are based on the detection of cancer associated ctDNA sequence mutations, or other DNA sequence effects associated with cancer. Other examples include methods based on detection of methylated DNA sequences associated with cancer or microRNA sequences associated with cancer. DNA abnormalities are characteristic of all cancer diseases. The DNA of cancer cells differs from that of healthy cells in many ways including, but not limited to, point mutations, methylation status, translocations, gene copy number, micro-satellite abnormalities and DNA strand integrity. Any of these mutations may be amenable to ctDNA testing.

Where cancer is detected at an early stage of disease, this is currently likely to be by a fecal test, an invasive endoscopy method, an invasive biopsy method, a potentially dangerous X-ray scanning method, an MRI scanning method, a cervical smear test or symptomatically. These methods all have disadvantages of invasiveness, unpleasantness, patient risk, patient compliance and/or high cost. Once detected, cancer is normally currently confirmed and diagnosed by immunohistochemistry (IHC) tests of tumour tissue removed at biopsy. Early detection of cancers leads to improved patient outcomes and financial benefits for healthcare providers in avoided expensive late stage hospital stays and treatments. Unfortunately, cancer currently often remains undetected until it has reached a late stage of disease development when it may have spread beyond the site of the primary tumour and treatment options are more limited, more expensive and less effective leading to poor patient outcomes. There is a clear unmet medical need for more and better cancer blood tests.

Patients diagnosed with cancer may present with late stage or metastatic cancer that has already spread beyond the organ in which the primary cancer developed. In many cases the site where the primary cancer originally developed is not clear and these cases are known as carcinomas of unknown primary (CUPs) or occult tumours. The identification of the primary tumour site in these cases is of great clinical relevance, because cancers are treated according to where they originally developed, even if they have spread to other parts of the body. This is particularly true for any targeted therapies given which should be targeted to the carcinoma of the primary site. For example, if a primary lung cancer has spread to the liver, it is a lung cancer with liver metastases or secondaries. It is not a liver cancer because the cancer cells are cancerous lung cells.

In many cases where a subject presents with metastatic cancer the site of the primary cancer is clear. However, in CUP the primary cancer cannot be determined. This may occur for a number of reasons, including for example, because the secondary cancer has grown very quickly, whilst the primary cancer is still small and not visible on scans, or because the primary cancer has disappeared, perhaps due to immune attack or sloughing off of a primary cancer (for example, a colorectal cancer may be sloughed from the bowel wall and passed out of the body with the feces).

Currently the primary tumour in patients presenting with metastatic disease is usually located by patient examination, patient symptoms, scanning and immunohistochemistry (IHC) tests of tumour tissue removed at biopsy. The primary tumour site can be identified or confirmed by IHC of tissue-specific transcription factors.

Circulating tumour DNA analysis, or other biomarker methods, may reveal the presence of a primary cancer in a subject without identifying the site of the cancer. This may lead to a similar situation as CUP (except there need not be any metastases present) where a subject is known or suspected to have cancer, but with no knowledge of the site of the cancer. For example, a cancer diagnosed using ctDNA sequence techniques may identify cancer associated gene mutations in the blood of a subject indicating the presence of a cancer without indicating the site of the cancer. A similar situation may occur for detection of cancer by circulating tumour cell techniques or other cancer detection methods which may not reveal the site of a tumour detected.

The human body comprises several hundred cell types. All of these cell types contain the same genome but widely different phenotypes and different functions in the body. This phenotypic diversity is due to the differential expression of the genome in different cell types. The control of differential gene expression is not entirely understood but the basic mechanisms include gene regulation by a number of interconnected epigenetic signals associated with the gene, including control of the chromatin packing as euchromatin or heterochromatin, control of nucleosome positioning and nuclease accessible sites, methylation of DNA and variation in the histone structure of the nucleosomes around which the DNA is wrapped and variation in transcription factor and transcription cofactor expression and interaction with the genes to which they bind.

The nucleosome is the basic unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller, 2007).

Normal cell turnover in adult humans involves the creation by cell division of some 10¹¹ cells daily and the death of a similar number, mainly by apoptosis. During the process of apoptosis chromatin is broken down into fragments including mononucleosomes and oligonucleosomes which are released from the cells. Under normal conditions these are removed and the level of circulating nucleosomes found in healthy subjects is low. Elevated levels are found in subjects with a variety of conditions including many cancers, auto-immune diseases, inflammatory conditions, stroke and myocardial infarction (Holdenrieder & Stieber, 2009).

Mononucleosomes and oligonucleosomes can be detected by Enzyme-Linked ImmunoSorbant Assay (ELISA) and several methods have been reported (Salgame et al, 1997; Holdenrieder et al, 2001; van Nieuwenhuijze et al, 2003). These assays typically employ an anti-histone antibody (for example, anti-H2B, anti-H3 or anti-H1, H2A, H2B, H3 and H4) as capture antibody and an anti-DNA or anti-H2A-H2B-DNA complex antibody as detection antibody. The correlation coefficient between ELISA results for circulating cell free nucleosomes levels and circulating DNA levels as measured by real time PCR (Polymerase Chain Reaction) has been reported to be r=0.531 in serum and r=0.350 in plasma (Holdenrieder et al, 2005).

Nucleosome ELISA methods are used in cell culture, primarily as a method to detect apoptosis (Salgame et al, 1997; Holdenrieder et al, 2001; van Nieuwenhuijze et al, 2003), and are also used for the measurement of circulating cell free nucleosomes in serum and plasma (Holdenrieder et al, 2001). Cell free serum and plasma nucleosome levels released into the circulation by dying cells have been measured by ELISA methods in studies of a number of different cancers to evaluate their use as a potential biomarker (Holdenrieder et al, 2001). Mean circulating nucleosome levels are reported to be high in most cancers studied. The highest circulating nucleosome levels were observed in lung cancer subjects. However, subjects with malignant tumours are reported to have serum nucleosome concentrations that varied considerably and some subjects with advanced tumour disease were found to have low circulating nucleosome levels, within the range measured for healthy subjects (Holdenrieder et al, 2001). Because of this and the variety of non-cancer causes of raised nucleosome levels, circulating nucleosome levels are not used clinically as a biomarker of cancer (Holdenrieder and Stieber, 2009).

The structure of nucleosomes can vary by Post Transcriptional Modification (PTM) of histone proteins and by the inclusion of variant histone proteins. PTM of histone proteins typically occurs on the tails of the eight core histones and common modifications include acetylation, methylation or ubiquitination of lysine residues as well as methylation of arginine residues and phosphorylation of serine residues. Histone modifications are known to be involved in epigenetic regulation of gene expression (Herranz and Esteller, 2007). The structure of the nucleosome can also vary by the inclusion of alternative histone isoforms or variants which are different gene or splice products and have different amino acid sequences. Histone variants can be classed into a number of families which are subdivided into individual types. The nucleotide sequences of a large number of histone variants are known and publicly available for example in the National Human Genome Research Institute NHGRI Histone DataBase (Mariño-Ramírez et al, 2011 and http://genome.nhgri.nih.gov/histones/complete.shtml), the GenBank (NIH genetic sequence) DataBase, the EMBL Nucleotide Sequence Database and the DNA Data Bank of Japan (DDBJ).

Histone variant and histone modification patterns present in healthy and diseased cells have been shown to differ in numerous (mostly immunohistochemical) studies (Herranz and Esteller, 2007). One disadvantage of immunohistochemical methods for clinical use is that tissue sample collection is invasive involving surgery or biopsy.

In addition to the epigenetic signalling mediated by nucleosome structure and position, control of gene expression in cells is also mediated by modifications to the structure of nucleosome associated DNA, for example by methylation of DNA (Herranz and Esteller, 2007). It has been known in the art for some time that DNA may be methylated at the 5 position of cytosine nucleotides to form 5-methylcytosine. Global DNA hypomethylation is a hallmark of cancer cells (Esteller, 2007 and Hervouet et al, 2010). Global DNA methylation can be studied in cells using immunohistochemistry techniques.

It has been known for many years that, in addition to nucleic acid and histone proteins, chromatin comprises a large number of non-histone proteins bound to its constituent DNA and/or histones (Yoshida and Shimura, 1972). These chromatin associated proteins are of a wide variety of types and have a variety of functions including transcription factors, transcription enhancement factors, transcription repression factors, histone modifying enzymes, DNA damage repair proteins and many more. The study of chromatin bound proteins has been carried out largely by Chromatin ImmunoPrecipitation (ChIP) methods. These methods are well known in the art but are complex, laborious and expensive.

In a typical ChIP method the cellular chromatin is cross-linked so that all the protein and nucleic acid components are covalently attached to each other. The chromatin is then sheared to form a preparation of mononucleosomes and oligonucleosomes. An antibody to the protein of interest is added to the sheared chromatin to immunoprecipitate those chromatin fragments containing the protein. The antibody is normally attached to a solid phase (e.g. plastic beads) to facilitate isolation of the chromatin complex containing the protein of interest. The cross-linking is then reversed and the protein is removed by digestion with a proteinase. The DNA associated with the chromatin complex is isolated and analysed to determine the DNA sequence, gene or locus associated with the particular protein binding using any of a variety of techniques including PCR followed by gel electrophoresis, DNA sequencing (ChIP-Seq) or DNA microarrays (ChIP-on-chip). These ChIP methods reveal the DNA sequences associated with chromatin bound histone proteins. Derivatives of the ChIP method have been developed to facilitate studies of the association of non-histone proteins with histones and nucleosomes including for example Histone Associated Assays (Ricke and Bielinsky, 2005).

Chromatin fragments containing non-histone proteins in the form of nucleosome-protein or chromatosome-protein adducts, have also been detected in the circulation as nucleosome-protein adducts. Example assays for detecting cell-free nucleosome-protein adducts are described in WO2013/084002.

In addition to other epigenetic regulation mechanisms, differential gene expression is regulated by variations in transcription factor expression and activity. Transcription factors are substances that contain a DNA Binding Domain (DBD) which binds to a specific gene sequence associated with the gene or genes whose expression is to be regulated. For example; the Androgen Receptor (AR) is a transcription factor that binds to a specific DNA sequence called the androgen response element (ARE) in chromosomes and upregulates or down-regulates the expression of androgen dependent genes that contain or are regulated by ARE. When transcription factors bind to their target gene sequence they may promote or suppress gene expression. Some transcription factors are expressed in all or most tissues. Other transcription factors may be differentially expressed in different tissues and are hence more tissue specific.

Tissue specific transcription factors have been shown by IHC to occur in the cells of certain primary cancer cells but not in others. For example, the transcription factor CDX2 occurs in gastroenterological cancer cells, particularly colorectal cancer cells, and its presence shown by IHC in tissue removed at biopsy or surgery may be used to identify a primary gastroenterological cancer.

Similarly, tissue specific transcription cofactors have been described including, for example, FHL2 which is reported to be a cofactor to the androgen receptor (AR) specific to prostate and heart tissues (Muller et al; 2000).

Ballare *et al.* (2013) discloses the requirement of nucleosomes for the optimal binding and function of progesterone receptor to progesterone-induced genes.

Snyder *et al.* (2016) discloses the use of transcription factor 'footprints' in short cell-free DNA fragments isolated from circulating blood plasma in the identification of the cell-type of origin.

Wittwer *et al.* (2013) discloses correlation of levels of circulating nucleosomes and immunogenic cell death markers with time to progression and overall survival in advanced pancreatic cancer patients undergoing chemotherapy.

Takaku *et al.* (2016) discloses the mechanism behind the transcription factor GATA3 functions as a pioneer transcription factor and its ability to bind inaccessible chromatin and induce chromatin remodelling or nucleosome sliding during the mesenchymal to epithelial transition.

Yang & Schwartz (2011) review epigenetic marks in the lung and their effects on gene expression in the lung associated with benign lung diseases and lung cancer.

Lin *et al.* (2011) discloses alterations in the mRNA levels for transcription factors GATA3, T-bet, RORyt and FOXP in peripheral blood mononuclear cells in patients with hepatocellular carcinoma.

We now report simple immunoassay methods for the direct estimation of transcription factor-nucleosome and transcription cofactor-nucleosome adducts in biological samples. By selection of tissue specific transcription factors and/or cofactors this method may be used as a non-invasive, or minimally invasive, blood test to detect a cancer or to determine or confirm the site of a primary tumour in a subject with metastatic disease. We have shown that such nucleosome adducts can be detected in serum samples and that they have use as biomarkers in disease.

### SUMMARY OF THE INVENTION

According to a first aspect of the invention there is provided the *in vitro* use of a tissue specific transcription factor-nucleosome adduct or transcription cofactor-nucleosome adduct as a biomarker in a biological fluid for the detection or diagnosis of a cancer in a subject wherein the tissue specific transcription factor-nucleosome adduct or transcription cofactor-nucleosome adduct is used to identify the site of development of the cancer in the subject.

According to a further aspect of the invention, there is provided a method for determining the site of development of a cancer in a subject which comprises the steps of:
(i) contacting a biological fluid sample obtained from the subject with a first binding agent which binds to nucleosomes or a component thereof, or a tissue specific transcription factor or cofactor adducted to a nucleosome;
(ii) contacting the nucleosomes or sample with a second binding agent which binds to a tissue specific transcription factor or cofactor adducted to a nucleosome when the first binding agent binds to the nucleosome or a component thereof, or said second binding agent binds to a nucleosome or component thereof when the first binding agent binds to a tissue specific transcription factor or cofactor adducted to a nucleosome; and
(iii) detecting or quantifying the binding of said second binding agent to the adducted tissue specific transcription factor or cofactor, nucleosomes or a component thereof in the sample;
wherein the presence or level of the tissue specific transcription factor or cofactor adducted to a nucleosome is used as an indicator of the site of development of said cancer.

According to a further aspect of the invention, there is provided a method for determining the site of development of a cancer in a subject which comprises the steps of:
(i) contacting a biological fluid sample obtained from the subject with a first binding agent which binds to nucleosomes or a component thereof;
(ii) contacting the nucleosomes or sample with a second binding agent which binds to a tissue specific transcription factor or cofactor adducted to a nucleosome; and
(iii) detecting or quantifying the binding of said second binding agent to the adducted tissue specific transcription factor or cofactor in the sample.

According to a further aspect of the invention, there is provided a method for determining the site of development of a cancer in a subject which comprises the steps of:
(i) contacting a biological fluid sample obtained from the subject with a first binding agent which binds to a tissue specific transcription factor or cofactor;
(ii) contacting the sample with a second binding agent which binds to nucleosomes or a component thereof; and
(iii) detecting or quantifying the binding of said second binding agent to nucleosomes or a component thereof in the sample.

According to a further aspect of the invention, there is provided a method for assessment of an animal or a human subject for suitability for a medical treatment which comprises the steps of:
(i) detecting or measuring a tissue specific transcription factor or cofactor adducted to a nucleosome in a biological fluid of the subject as defined in the method as described herein; and
(ii) using the type of tissue specific transcription factor or cofactor adducted to a nucleosome detected to determine a suitable treatment for the subject.

According to a further aspect of the invention, there is provided a method for monitoring a treatment of an animal or a human subject which comprises the steps of:
(i) detecting or measuring a tissue specific transcription factor or cofactor adducted to a nucleosome in a biological fluid of the subject as defined in the method as described herein;
(ii) repeating the detection or measurement of the tissue specific transcription factor or cofactor adducted to a nucleosome in a biological fluid of the subject on one or more occasions; and
(iii) using any changes in the level of tissue specific transcription factor or cofactor adducted to a nucleosome detected as a parameter for any changes in the condition of the subject.

### BRIEF DESCRIPTION OF THE FIGURES

**Figure 1****:** Circulating cell free nucleosome bound GATA 3 adduct ELISA results for samples taken from a healthy subject, 3 subjects diagnosed with breast cancer, 2 subjects diagnosed with bladder cancer and a negative control sample.
**Figure 2****:** Circulating cell free nucleosome bound TTF-1 adduct ELISA results for samples taken from a healthy subject, 3 subjects diagnosed with thyroid cancer, 2 subjects diagnosed with lung cancer and a negative control sample.
**Figure 3****:** Circulating cell free nucleosome bound TTF-1 adduct ELISA results for samples taken from 3 healthy subjects, 1 subject diagnosed with thyroid cancer, 4 subjects diagnosed with lung cancer, 3 subjects diagnosed with rectal cancer, 3 subjects diagnosed with colon cancer, 3 subjects diagnosed with breast cancer and a negative control sample.
**Figure 4****:** Circulating cell free nucleosome bound CDX2 ELISA adduct results for samples taken from 3 healthy subjects, 1 subject diagnosed with thyroid cancer, 4 subjects diagnosed with lung cancer, 3 subjects diagnosed with rectal cancer, 3 subjects diagnosed with colon cancer, 3 subjects diagnosed with breast cancer and a negative control sample.

### DETAILED DESCRIPTION OF THE INVENTION

According to a first aspect of the invention, there is provided the in vitro use of a tissue specific transcription factor or cofactor nucleosome adduct as a biomarker in a biological fluid for the detection or diagnosis of a cancer in a subject wherein the tissue specific transcription factor-nucleosome adduct or transcription cofactor-nucleosome adduct is used to identify the site of development of the cancer in the subject.

In one embodiment, the subject has not previously been diagnosed with cancer (i.e. is apparently healthy). For example, the cancer is detected in an apparently healthy person in a screening test. In one embodiment, the subject has been identified with symptoms associated with cancer, i.e. the cancer is detected in a person with symptoms of disease. The symptoms may be suggestive of cancer or suggestive of malfunction of a particular tissue or organ. Such symptoms can include pain, unusual bleeding, an unusual lump or swelling, a persistent cough, shortness of breath and/or unexplained weight loss.

The present invention has utility in patients who have already been diagnosed with a primary cancer without knowledge of the location of the cancer, for example using a ctDNA test, a circulating tumour cell test or symptomatically. Therefore, according to one embodiment of the invention, there is provided the use of a tissue specific transcription factor or cofactor nucleosome adduct as a biomarker in a biological fluid for the identification or diagnosis of the site of development of a cancer in a subject who has previously been diagnosed with cancer.

We have found that tissue specific transcription factors or cofactors can be detected on cell-free nucleosomes, and can therefore be used to detect a cancer and/or to identify the origin of a cancer in a patient, such as in patients who have previously been diagnosed with cancer but without knowledge of its site of development. The identification of the development site of a cancer is critical in order to choose the appropriate therapy and therefore improve a patient's prognosis and treatment.

In one embodiment, the cancer is a primary cancer, i.e. the place where a cancer starts/originates in the body. In an alternative embodiment, the cancer is a secondary cancer, i.e. a metastasis once the cancer cells have spread to another part of the body.

In one embodiment, the subject is a subject with metastatic cancer of unknown primary (CUP). In this embodiment, the subject may have been diagnosed with cancer by identifying a metastasis, but it is still important to locate the primary cancer in order to provide the appropriate treatment.

In one embodiment, the subject was previously diagnosed with cancer using a circulating nucleic acid method, for example, using ctDNA, RNA, miRNA or other circulating blood nucleic acid based methods which detect cancer *per se*. In a further embodiment, the subject was previously diagnosed with cancer using circulating tumour DNA (ctDNA) as a biomarker. In one embodiment, the subject was previously diagnosed with cancer by detecting circulating tumour cells. In these embodiments, the cancer is detected but with insufficient or no information on the original location of the cancer in a subject.

According to a further aspect of the invention there is provided the use of a tissue specific transcription factor-nucleosome adduct as a biomarker in a biological fluid for the identification or diagnosis of the site, position or organ location of a primary cancer in a subject with metastatic disease. We have shown that three such adducts containing TTF-1, CDX2 or GATA 3, which are expressed in thyroid or lung cancer tissue (TTF-1), gastroenterological cancer tissue (CDX2) and breast cancer tissue (GATA 3) respectively, are present in the circulation of subjects with cancer and that their presence or level can be used to indicate that the patient has a primary thyroid, colorectal or breast cancer respectively and that these tissue specific transcription factor-nucleosome adducts are not commonly present (or present at low levels) in the circulation of subjects with other cancers or healthy subjects.

In the literature it is reported that TTF-1 is expressed in most lung cancer tissue samples as well as most thyroid cancer tissue samples. Colorectal cancer tissue is sometimes elevated in tissue TTF-1 expression. Tissue assays for TTF-1 can therefore be used to identify CUP tumors as being of thyroid or lung primary origin but some other cancers including colorectal cancer are possible "false positive" results that may wrongly be identified as a lung or thyroid cancer. We have found that circulating nucleosome bound TTF-1 adduct levels are elevated in the blood of subjects with thyroid tumors and less elevated in subjects with lung cancer. Surprisingly, we also found that circulating nucleosome bound TTF-1 adduct levels were elevated in the blood of all subjects diagnosed with colorectal (colon or rectal) cancer. This indicates that the tissue specificity of cell free nucleosome-transcription factor adduct levels in body fluids may not in all cases, be the same as the specificity of transcription factor expression levels in cancer tissues removed at biopsy.

Similar tissue specific transcription factor expression is known to occur for many other cancers (Kandalaft and Gown, 2015) and it will be clear to those skilled in the art that analogous nucleosome adduct assays for appropriate tissue specific transcription factor-nucleosome adducts can be used to identify the cancer of unknown primary site for the wide range of cancers where such transcription factors have already been identified. This includes for example, without limitation, GATA 3, CDX2, TTF-1, PAX8, WT1, NKX3.1, P63 (TP63) or P40 and/or any cancer where any such tissue specific transcription factor expression can be identified. The method of the invention has clinical utility in any situation where a subject may have an undetected or unknown cancer or is known to have a cancer, but the site of development of the primary tumour is unknown, including in cases of CUP or where cancer is detected without knowledge of its site of development. This latter situation may arise, for example, where cancer detection methods based on ctDNA, RNA or other circulating blood nucleic acid based methods which detect cancer *per se* are used or where circulating tumour cells of unknown primary origin are detected. There are a wide variety of circulating nucleic acid methods. Some are based on the detection of cancer associated ctDNA sequence mutations, or other DNA sequence effects associated with cancer. Others are based on detection of microRNA sequences associated with cancer, methylated DNA sequences associated with cancer and others. DNA abnormalities are characteristic of all cancer diseases. The DNA of cancer cells differs from that of healthy cells in many ways including, but not limited to, point mutations, methylation status, translocations, gene copy number, micro-satellite abnormalities and DNA strand integrity. However, genetic mutations are characteristic of all cancers and their presence may not provide information regarding the nature of the primary tumor. As an illustrative example; P53 gene mutations are common in cancer and their presence in ctDNA may be used as a biomarker for cancer detection. However, because P53 mutations are present in a wide variety of cancers their presence will be uninformative on the nature of the primary cancer. It will be clear to those skilled in the art that the present invention is useful for determining the site of cancer development where cancer *per se* has been detected by another method using any biomarker or for symptomatic reasons, regardless of that method of detection. Therefore, the present invention is a useful secondary test in patients previously diagnosed with cancer but who are unaware of the origin, site or location of the disease.

References to "transcription factor" refer to proteins that bind to DNA and regulate gene expression by promoting (i.e. activators) or suppressing (i.e. repressors) transcription. Transcription factors contain one or more DNA-binding domains (DBDs), which attach to specific sequences of DNA adjacent to the genes that they regulate.

The term "tissue specific transcription factor" as described herein refers to a transcription factor that is always or commonly expressed in certain tissues or cancers whilst being rarely or never expressed in other tissues or cancers. Such tissue specific transcription factors may only be expressed in a limited number of tissue or cancer types, such as only one, two, three, four or five tissue or cancer types. The presence or level of the cell free nucleosome-transcription factor adduct in the circulation indicates cell death of cells expressing that transcription factor and its tissue specificity can be used as a biomarker to indicate the tissue of origin as the primary site of tumour development.

The term "cofactor" or "transcription factor cofactor" as described herein refers to proteins that modulate the effects of transcription factors. Many transcription factors, bind their target DNA sequence only in the presence of these collaborating cofactors which modulate binding and are sometimes involved in the recruitment of the preinitiation complex and RNA polymerase. References to embodiments involving transcription factors, as used herein, may equally apply to cofactors.

Furthermore, tissue specific combinations of transcription factors and/or transcription cofactors have been described wherein transcription factors, which may not be tissue specific in terms of their own expression, regulate tissue specific gene expression through combinatorial transcription factor control that is tissue specific. Thus, binding of two or more transcription factors or cofactors, whose individual expression may not be tissue specific, may in fact regulate the expression of a tissue specific gene, where expression of the gene requires the presence of both or multiple transcription factors or cofactors. Therefore, references to "tissue specific" transcription factors and cofactors include combinations of transcription factors and/or cofactors which work in concert to achieve tissue specificity, i.e. in one embodiment, the aspects described herein comprise detecting a combination of transcription factors and/or cofactors which is tissue specific.

The term "biomarker" means a distinctive biological or biologically derived indicator of a process, event, or condition. Biomarkers can be used in methods of differential diagnosis of cancer, e.g. prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

According to a further aspect of the invention there is provided the use of a tissue specific transcription factor-nucleosome or cofactor-nucleosome adduct as a biomarker in blood for the identification or diagnosis of the site of a primary cancer which was detected in a subject without knowledge of the site of that cancer. In one embodiment, the presence of a cancer is suspected in a subject for symptomatic reasons (i.e. based on patient symptoms) and the invention is used to confirm the presence and/or site, position or organ location of the cancer.

The present invention is aimed at detection of tissue specific transcription factors or cofactors which are bound to nucleosomes in a body fluid. This can be done by means of taking a sample of a body fluid from a subject, and performing a double antibody ELISA test in which one antibody is directed to bind to nucleosomes and the other is directed to bind to the tissue specific transcription factor or cofactor bound or adducted to a nucleosome. However, the antibody directed to bind to the nucleosome need not be directed to the whole nucleosome complex but may be directed to a component part of the nucleosome. In this embodiment of the invention the antibody employed to bind to the nucleosome may be directed to bind any component part of a nucleosome including, for example to a particular histone, histone modification, histone variant or isoform or to DNA or to a particular nucleotide, such as 5-methylcytosine. Similarly, the antibody employed to bind to the tissue specific transcription factor may be directed to bind any component part or domain of the transcription factor. Where the transcription factor is a protein complex consisting of multiple proteins any of these may similarly be targeted by the antibody. According to a further aspect of the invention there is provided a method for determining the site of development of a cancer in a subject which comprises the steps of:
(i) contacting a biological sample obtained from the subject with a first binding agent which binds to nucleosomes or a component thereof, or a tissue specific transcription factor or cofactor adducted to a nucleosome;
(ii) contacting the nucleosomes or sample with a second binding agent which binds to a tissue specific transcription factor or cofactor adducted to a nucleosome when the first binding agent binds to the nucleosome or a component thereof, or said second binding agent binds to a nucleosome or component thereof when the first binding agent binds to a tissue specific transcription factor or cofactor adducted to a nucleosome; and
(iii) detecting or quantifying the binding of said second binding agent to the adducted tissue specific transcription factor or cofactor, nucleosomes or a component thereof in the sample;
wherein the presence or level of the tissue specific transcription factor or cofactor adducted to a nucleosome is used as an indicator of the site of development of said cancer.

According to one aspect of the invention there is provided a method for determining the site of development of a cancer in a subject which comprises the steps of:
(i) contacting a biological sample obtained from the subject with a first binding agent which binds to nucleosomes or a component thereof;
(ii) contacting the nucleosomes or sample with a second binding agent which binds to a tissue specific transcription factor or cofactor adducted to a nucleosome; and
(iii) detecting or quantifying the binding of said second binding agent to the adducted tissue specific transcription factor or cofactor in the sample;
wherein the presence or level of the tissue specific transcription factor or cofactor adducted to a nucleosome is used as an indicator of the site of development of said cancer.

It will be clear to those skilled in the art that the detection binding agent may be selected to be directed to bind either to the adducted tissue specific transcription factor or cofactor or to the nucleosome or a component part of the nucleosome.

Therefore, according to a further aspect of the invention there is provided a method for determining the site of development of a cancer in a subject which comprises the steps of:
(i) contacting a biological sample obtained from the subject with a first binding agent which binds to a tissue specific transcription factor or cofactor adducted to a nucleosome;
(ii) contacting the nucleosomes or sample with a second binding agent which binds to nucleosomes or a component thereof; and
(iii) detecting or quantifying the binding of said second binding agent to nucleosomes or a component thereof in the sample;
wherein the presence or level of the tissue specific transcription factor or cofactor adducted to a nucleosome is used as an indicator of the site of development of said cancer.

The methods of the present invention may also be used in a subject who has previously been diagnosed with cancer.

As noted above tissue specificity may be conferred on transcription factors by cooperative binding with one or multiple other transcription factors or cofactors and these multiple cofactors may be closely co-located at a site in the genome (e.g. see Zhang & Glass, 2013; and Yu et al, 2006). This means that chromatin digestion on cell death will lead to chromatin fragments containing multiple transcription factors. Such chromatin fragments may include multiple transcription factors in the presence or absence of a nucleosome. Chromatin fragments including multiple transcription factors can be used in further aspects of the invention.

Whilst co-location of transcription factors and cofactors often occurs due to co-location of their respective DNA binding sites in the genome, co-location of transcription factors and cofactors whose binding sites are further apart may also occur due to looping in the DNA where more distal sites are brought together for gene regulation. It will be clear to those skilled in the art that chromatin fragments may also contain tissue specific combinations of transcription factors and cofactors resulting from such loops.

The term "chromatin fragment" as used herein refers to a complex of proteins and nucleic acid whose origin lies in the chromosome of a cell. A fragment of chromatin may contain a nucleosome and/or associated DNA and/or any of a huge variety of non-histone chromatin associated proteins in a multi-protein-nucleic acid complex. Some examples of non-histone chromatin associated proteins include transcription factors, cofactors, co-activators, co-repressors, RNA polymerase moieties, elongation factors, chromatin remodelling factors, mediators, STAT moieties, upstream binding factor (UBF) and others.

It will be understood that chromatin fragments that include a (tissue specific) transcription factor or cofactor that confers tissue specificity are themselves tissue specific. Similarly, chromatin fragments that include a tissue specific combination of two or more transcription factors or cofactors that confer tissue specificity are themselves tissue specific. Therefore, the term "tissue specific chromatin fragment" as described herein refers to a chromatin fragment that includes a tissue specific transcription factor or cofactor, or includes a tissue specific combination of two or more transcription factors or cofactors.

Simultaneous binding of two or more transcription factors or cofactors to a single gene or gene promoter locus may be specific to a single tissue or to a limited number of tissues. Where such tissue specific simultaneous binding of transcription factors and/or cofactors occurs, the binding motifs are usually in close proximity and separated by less than 200 base pairs.

Such collaborating factors may confer tissue specificity to the actions of many signal-dependent transcription factors that are themselves widely expressed. Examples include members of diverse families of transcription factors that are activated by extracellular signals through cell surface receptors including, without limitation, nuclear hormone receptors, STAT transcription factors, NF-κB family members, CREB and others. Thus, widely expressed cell surface receptors may regulate a variety of different genes in different tissues, each in a tissue specific manner. Reported examples include the androgen receptor regulation of transcription of prostate specific genes in collaboration with the transcription factor FoxA1, transcription of kidney specific genes in collaboration with the transcription factor Hnf4α and transcription of epididymis specific genes in collaboration with the transcription factor AP-2α (Pihlajamaa, 2014). Similar examples have been reported for other nuclear hormone receptors in Levy et al, 2007; Zhao et al, 2010; and Zhang & Glass, 2013;

A large number of tissue specific combinations of transcription factors and cofactors suitable for use in the invention are known and may be found in publicly available data bases including, for example the Tissue Specific Gene Expression and Regulation (TiGER) database developed by the Bioinformatics Laboratory at the Wilmer Eye Institute of Johns Hopkins University. Three example pairs of transcription factors or cofactors each with tissue specificity for a variety of tissues (including Peripheral Nervous System (PNS) and mammary gland (Mammary)) are shown below:

| | | | |
|---|---|---|---|
| Bladder | ARNT:SREBP1 | MAX:SREBP1 | SREBP1:MYC/MAX |
| Blood | ELF1:PEA3 | ETF:NRF1 | PEA3:PU.1 |
| Bone | EF-C:MIF1 | EF-C:RFX1 | GATA-3:MIF1 |
| Bone marrow | ETF:NRF1 | MAX:SREBP1 | NRF1:STAT3 |
| Brain | AP2alpha:ETF | C/EBP:PBX1 | ETF:LBP1 |
| Cervix | CREB:NRF1 | ELK1:NRF1 | ETF:NRF1 |
| Colon | AFP1:HNF1 | CDP:FOXO4 | CDP:HNF1 |
| Eye | CHX10:CRX | CHX10:GATA6 | CRX:PITX2 |
| Heart | AP1:MEF2 | AP1:RSRFC4 | FOXJ2:POU3F2 |
| Kidney | CRX:HNF1 | GCNF:HNF1 | COUP-TF/HNF4:HNF1 |
| Larynx | AP1:NFE2 | AP1:TCF11/MAFG | BACH1:NFE2 |
| Liver | CDP:HNF1 | C/EBPgamma:HNF1 | E4BP4:HNF1 |
| Lung | ETF:MYC/MAX | ETF:LBP1 | ETF:TBP |
| Lymph node | c-Ets-1:c-Ets-2 | ELK1:PU.1 | ICSBP:c-Ets-2 |
| Mammary | E2F:SRY | ETF:TAX/CREB | ETF:ZID |
| Muscle | AP-4:MEF2 | MEF2:MYOD | MEF2:RSRFC4 |
| Ovary | AP2alpha:VDR | CREB:MAZ | DBP:VDR |
| Pancreas | ATF:HEB | ATF:NF-muE1 | ATF:NRL |
| PNS | CREB:RSRFC4 | HNF1:SRY | MYOD:OCT1 |
| Placenta | ATF1:CHX10 | ATF1:LHX3 | ATF:CHX10 |
| Prostate | AFP1:LHX3 | CART1:LHX3 | C/EBPγ:LHX3 |
| Skin | AREB6:ALX4 | AREB6:ARP-1 | AREB6:E47 |
| Intestine | CART1:LHX3 | HNF1:LHX3 | HNF1:NKX6-2 |
| Soft tissue | C/EBPγ:FOXO4 | FOXO1:SF1 | FOXO4:TBP |
| Spleen | E12:c-Ets-1 | GCM:NFKB1 | LBP1:MYOD |
| Stomach | AP2γ:ETF | AREB6:NFE2 | ER:HIF1 |
| Testis | AP2:NRF1 | EGR1:NRF1 | EGR3:NRF1 |
| Thymus | ATF:TAX/CREB | c-Ets-1:c-Ets-2 | ETF:NRF1 |
| Tongue | ATF:CREB | ATF:CREBP1 | CREBP1:CREB |
| Uterus | E4BP4:POU1F1 | E4BP4:POU3F2 | NKX6-2:POU3F2 |

It will be clear to those skilled in the art that tissue specific cell free transcription factors and/or cofactors, and combinations thereof, in biological fluids may be regarded as tissue specific biomarkers of cellular or chromatin origin whether or not nucleosome or DNA bound.

It will be clear to those skilled in the art that a wide variety of immunochemical and other analysis methods may be used to measure tissue specific chromatin fragments including transcription factor- and/or cofactor-nucleosome adducts in conjunction with the present invention. A variety of molecular biological methods are also suitable for use in conjunction with the present invention, including for example without limitation Chromatin Immunoprecipitation based methods (ChIP) or other methods for the analysis of chromatin material. Non-immunochemical methods include any chromatographic or mass spectrometry method and any method involving disassociation of a tissue specific transcription factor-nucleosome adduct and measurement of the disassociated tissue specific transcription factor by immunochemical, chromatographic, mass spectrophotometric or other means. Methods for disassociating protein complexes or adducts and protein-nucleic acid complexes or adducts are well known in the art, for example without limitation; by exposing said adducts to acidic or basic pH media or to high salt concentrations, or by mechanical or sonication methods or by biological digestion or other enzyme based methods.

Transcription factors that bind directly to their target nucleosomal DNA are referred to in the art as pioneering transcription factors. Many transcription factors, however, bind their target DNA sequence only in the presence of collaborating cofactors which modulate binding and may also confer further tissue specificity. For example; FoxA1 is a cofactor required for Androgen Receptor (AR) binding to the androgen response element (ARE) in prostate tissue and for Estrogen Receptor (AR) binding to the estrogen response element (ERE) in breast cancer. Thus, in one embodiment of the invention a cell free nucleosome bound cofactor (of a transcription factor) is measured and used to identify the site of development of a cancer. The transcription factor may, or may not, also be nucleosome bound with the cofactor. The cell free nucleosome bound cofactor may be measured in the presence or absence of the transcription factor.

In one embodiment, the tissue specific cofactor is FoxA1. If the cofactor is FoxA1, the site of development of a cancer may be selected from: Breast and Prostate.

In a preferred embodiment, the tissue specific transcription factor is selected from: GATA 3, CDX2, TTF-1, PAX8, WT1, NKX3.1, P63 (TP63) or P40. Table 1 lists the tumours associated with these transcription factors in cancer tissue.

**TABLE 1: Examples of tissue specific transcription factors and their associated tumours in cancer tissue**

| **Transcription Factor** | **Associated tumours** |
|---|---|
| GATA 3 | Breast, Salivary gland, Transitional cell carcinomas, Skin adnexal tumours |
| CDX2 | Colorectal, Pancreatic, Gastric carcinomas |
| TTF-1 | Lung adenocarcinoma, Thyroid, Neuroendocrine carcinomas |
| PAX8 | Gynaecological, Thyroid, Renal cell carcinoma |
| WT1 | Ovarian, Mesothelioma |
| NKX3.1 | Prostate |
| P63 (TP63) | Squamous cell carcinomas, Transitional cell carcinomas, Thymoma, Salivary gland, Trophoblastic tumours |
| P40 | Squamous cell carcinomas, Transitional cell carcinomas, Thymoma, Salivary gland, Trophoblastic tumours |

The examples in Table 1 are not exhaustive. The tissue specificity of nucleosome transcription factor adduct levels detected in body fluids by the methods of the invention may sometimes differ from that of the transcription factor expression levels in cancer tissue.

In one embodiment, said tissue specific transcription factor is GATA 3 and the site of development of a cancer is selected from: Breast, Salivary gland, Transitional cell carcinomas and Skin adnexal tumours.

In one embodiment, said tissue specific transcription factor is CDX2 and the site of development of a cancer is selected from: Colorectal and Gastric carcinomas.

In one embodiment, said tissue specific transcription factor is TTF-1 and the site of development of a cancer is selected from: Lung adenocarcinoma, Colorectal carcinoma, Thyroid and Neuroendocrine carcinomas.

In one embodiment, said tissue specific transcription factor is PAX8 and the site of development of a cancer is selected from: Gynaecological, Thyroid and Renal cell carcinoma.

In one embodiment, said tissue specific transcription factor is WT1 and the site of development of a cancer is selected from: Ovarian and Mesothelioma.

In one embodiment, said tissue specific transcription factor is NKX3.1 and the site of development of a cancer is prostate.

In one embodiment, said tissue specific transcription factor is P63 (TP63) and the site of development of a cancer is selected from: Squamous cell carcinomas, Transitional cell carcinomas, Thymoma, Salivary gland and Trophoblastic tumours.

In one embodiment, said tissue specific transcription factor is P40 and the site of development of a cancer is selected from: Squamous cell carcinomas, Transitional cell carcinomas, Thymoma, Salivary gland and Trophoblastic tumours.

The method can be performed using an antibody directed to the nucleosome itself in combination with an antibody directed to bind to the tissue specific transcription factor adducted to the nucleosome or using an antibody directed to a component of a nucleosome, again in combination with an antibody directed to bind to the tissue specific transcription factor adducted to the nucleosome.

In one embodiment, the binding agent which binds to nucleosomes or a component thereof, is directed to bind to a particular histone, histone modification, histone variant or isoform, or a particular nucleotide.

The term "binding agent" refers to ligands or binders, such as naturally occurring or chemically synthesised compounds, capable of specific binding to a biomarker (i.e. a nucleosome or tissue specific transcription factor adducted to the nucleosome). A ligand or binder according to the invention may comprise a peptide, an antibody or a fragment thereof, or a synthetic ligand such as a plastic antibody, or an aptamer or oligonucleotide, capable of specific binding to the biomarker. The antibody can be a polyclonal or monoclonal antibody or a fragment thereof capable of specific binding to the target. A ligand or binder according to the invention may be labelled with a detectable marker, such as a luminescent, fluorescent, enzyme or radioactive marker; alternatively, or additionally a ligand according to the invention may be labelled with an affinity tag, e.g. a biotin, avidin, streptavidin or His (e.g. hexa-His) tag. In one embodiment, the binding agent is selected from: an antibody, an antibody fragment or an aptamer. In a further embodiment, the binding agent used is an antibody. The terms "antibody", "binding agent" or "binder" are used interchangeably herein.

In one embodiment, the sample is a biological fluid (which is used interchangeably with the term "body fluid" herein). The fluid sample may be any biological fluid sample obtained from a subject including, without limitation, cerebrospinal fluid (CSF), whole blood, blood serum, plasma, menstrual blood, endometrial fluid, urine, saliva, or other bodily fluid (stool, tear fluid, synovial fluid, sputum), breath, e.g. as condensed breath, or an extract or purification therefrom, or dilution thereof. Biological samples also include specimens obtained from a live subject, or taken post-mortem. The samples can be prepared, for example where appropriate diluted or concentrated, and stored in the usual manner. In a further embodiment, the biological fluid sample is selected from: blood or serum or plasma. It will be clear to those skilled in the art that the detection of nucleosome adducts in a body fluid has the advantage of being a minimally invasive method that does not require biopsy.

In one embodiment, the subject is a mammalian subject. In a further embodiment, the subject is selected from a human or animal (such as mouse) subject.

In one embodiment, the nucleosome is a cell free mononucleosome or oligonucleosome.

It will be understood that the uses and methods of the invention may be performed *in vitro,* and/or *ex vivo.*

In one embodiment of the invention, the presence or level of one or more tissue specific transcription factors in a chromatin fragment or adducted to a nucleosome in a sample is used to determine the optimal treatment regime for a subject in need of such treatment. It will be understood by a person skilled in the art that the tissue specific chromatin fragment or transcription factor-nucleosome adduct can be used to identify the site, position and/or organ location of the cancer which can be used to determine the optimal treatment, i.e. based on the origin/location of the cancer.

According to a further aspect of the invention there is provided a method for assessment of an animal or a human subject for suitability for a medical treatment which comprises the steps of:
(i) detecting or measuring one or more tissue specific transcription factors or cofactors in a chromatin fragment or adducted to a nucleosome in a biological fluid of the subject; and
(ii) using the type of tissue specific transcription factor or cofactor in a chromatin fragment or adducted to the nucleosome detected to determine a suitable treatment for the subject.

According to a further aspect of the invention there is provided a method for monitoring a treatment of an animal or a human subject which comprises the steps of:
(i) detecting or measuring one or more tissue specific transcription factors or cofactors in a chromatin fragment or adducted to a nucleosome in a biological fluid of the subject;
(ii) repeating the detection or measurement of the one or more tissue specific transcription factors or cofactors in a chromatin fragment or adducted to a nucleosome in a biological fluid of the subject on one or more occasions; and
(iii) using any changes in the level of the one or more tissue specific transcription factors or cofactors in a chromatin fragment or adducted to a nucleosome detected as a parameter for any changes in the condition of the subject.

A change in the level of the tissue specific chromatin fragment or transcription factor- or cofactor-nucleosome adduct in the test sample relative to the level in a previous test sample taken earlier from the same test subject may be indicative of a beneficial effect, e.g. stabilisation or improvement, of said therapy on the disorder or suspected disorder. Furthermore, once treatment has been completed, the method of the invention may be periodically repeated in order to monitor for the recurrence of a disease.

In one embodiment, the tissue specific chromatin fragment or transcription factor nucleosome adduct is detected or measured as one of a panel of measurements. For example, the tissue specific transcription factor nucleosome adduct could be detected with other biomarkers which are specific for the same tissue, for example in combination with the tissue specific markers described in Kandalaft and Gown, 2015.

In a further embodiment of the invention there is provided a method for detecting or diagnosing the type of a cancer disease to assess the optimal cancer type to which a drug or other treatment option should be targeted, comprising testing a sample taken from a subject in whom a cancer has been diagnosed for the presence or level of a tissue specific transcription factor adducted to a nucleosome as part of a panel of tests.

Thus, such a panel of tests may consist, for example, of two or more or multiple measurements of nucleosome adducts containing different tissue specific transcription factors.

Efficient differential diagnosis of cancer types and monitoring methods provide very powerful "patient solutions" with the potential for improved prognosis, by establishing the correct diagnosis, allowing rapid identification of the most appropriate treatment (thus lessening unnecessary exposure to harmful drug side effects), and reducing relapse rates.

It will be understood that identifying and/or quantifying can be performed by any method suitable to identify the presence and/or amount of a specific protein in a biological sample from a patient or a purification or extract of a biological sample or a dilution thereof. In methods of the invention, quantifying may be performed by measuring the concentration of the biomarker in the sample or samples. Biological samples that may be tested in a method of the invention include those as defined hereinbefore. The samples can be prepared, for example where appropriate diluted or concentrated, and stored in the usual manner.

Identification and/or quantification of biomarkers may be performed by detection of the biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand or binder may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT^{®} (Applied Biosystems, CA, USA), or iTRAQ^{®} (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Methods of differential diagnosis of cancer or monitoring according to the invention may comprise analysing a sample by SELDI TOF or MALDI TOF to detect the presence or level of the biomarker. These methods are also suitable for prognosis, monitoring the results of therapy, identifying patients most likely to respond to a particular therapeutic treatment, for drug screening and development, and identification of new targets for drug treatment.

Identifying and/or quantifying the analyte biomarkers may be performed using an immunological method, involving an antibody, or a fragment thereof capable of specific binding to the biomarker. Suitable immunological methods include sandwich immunoassays, such as sandwich ELISA, in which the detection of the analyte biomarkers is performed using two antibodies which recognize different epitopes on a analyte biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format.

The immunoassays of the invention include immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays and competitive immunoassay methods including labelled antigen and labelled antibody competitive immunoassay methods with a variety of label types including radioactive, enzyme, fluorescent, time-resolved fluorescent and particulate labels. Some immunoassays employ methods to detect antibody binding directly without the use of any labelled moieties. All of said immunoassay methods are well known in the art, see for example Salgame et al, 1997 and van Nieuwenhuijze et al, 2003.

Also disclosed herein is a kit for the detection of a tissue specific transcription factor or cofactor adducted to a nucleosome which comprises a ligand or binder specific for the tissue specific transcription factor and another ligand or binder specific for a nucleosome or component part thereof, together with instructions for use of the kit in accordance with the methods described herein.

Further disclosed is a kit for the detection of a tissue specific chromatin fragment which comprises a ligand or binder specific for a first transcription factor or cofactor and another ligand or binder specific for a second transcription factor or cofactor, together with instructions for use of the kit in accordance with the methods described herein.

We used an anti-histone antibody as the detection antibody for the assays described herein, in combination with an appropriate specific anti-tissue specific transcription factor antibody as capture antibody. We have used the assays to show that nucleosome adducts containing specific tissue specific transcription factors can be measured in blood samples taken from subjects with cancer and are discriminating for use as non-invasive or minimally invasive biomarkers.

It will be clear to those skilled in the art that the uses and methods of the present invention can be used to detect or measure any tissue specific transcription factor or cofactor that may be attached or bound to nucleosomes.

We conclude that the method of the present invention is a successful method for the detection and measurement of tissue specific chromatin fragments and tissue specific transcription factor-nucleosome adducts, and that this method is a superior method for the non-invasive detection of a cancer in a subject and/or for the identification of a primary tumour detected by another cancer detection method (e.g. by ctDNA sequence methods) or in CUP.

The invention will now be described in further detail with reference to the following, non-limiting Examples.

### EXAMPLE 1

Serum samples were taken from 1 healthy subject, 2 subjects with breast cancer and 2 subjects with urinary urothelial bladder cancer (a transitional cell cancer known to give positive results in GATA 3 cancer tissue expression tests). A commercially available 96 well research use only ELISA kit for GATA 3 was purchased which included a solid phase antibody for GATA 3 transcription factor coated to the microtiter wells. GATA 3 is a tissue specific transcription factor expressed in all or most biopsy material samples taken from breast cancers, that is also positive in transitional cell carcinomas and skin adnexal tumours but not in most samples from other cancers. The solid phase GATA 3 antibody was used together with a biotinylated anti-nucleosome antibody, in a double antibody ELISA for cell free nucleosome bound GATA 3 as follows: serum sample (50µl) and assay buffer (50µl) were added to an anti-GATA 3 antibody coated well and incubated at room temperature (approximately 20°C) with shaking for 2.5 hours. The sample was then removed and biotinylated anti-nucleosome antibody (100µl) was added and incubated 1.5 hours. The biotinylated antibody solution was removed and the well was washed 3 times with wash buffer (200µl). A streptavidin-HRP conjugate solution was added to the well and incubated 30 minutes. The well washed a further 3 times and HRP substrate solution (100µl of 2,2'-Azinobis [3-ethylbenzothiazoline-6-sulfonic acid]-diammonium salt solution) was added and incubated for 20 minutes. A STOP solution (50µl) was added and the Optical Density (OD) of the well determined at 405nm.

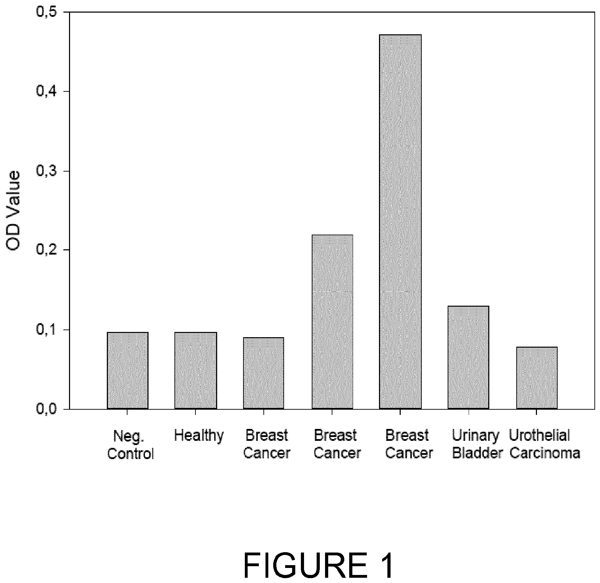

A higher ELISA signal, and a higher level of circulating cell free nucleosome-GATA 3 adduct, was observed for samples taken from 2 of the 3 subjects with breast cancer than from the healthy subject or the negative control. One of two subjects with a transitional cell cancer also had a raised OD level over the healthy subject. The results are shown in Figure 1 and illustrate that a serum nucleosome-GATA 3 adduct assay can be used to detect a cancer and/or to identify a cancer of unknown primary site of development where it is a breast cancer and that the nucleosome-GATA 3 adduct can be used as a biomarker for this purpose.

### EXAMPLE 2

Serum or samples were taken from 2 subjects with thyroid cancer, 3 subjects with lung cancer and 1 healthy subject. A commercially available 96 well research use only ELISA kit for TTF-1 was purchased which included a solid phase antibody for TTF-1 (thyroid transcription factor 1) coated to the microtiter wells. TTF-1 is a tissue specific transcription factor expressed in all or most biopsy material samples taken from thyroid and lung cancers, but not in most samples from other cancers. The solid phase TTF-1 antibody was used together with a biotinylated anti-nucleosome antibody, in a double antibody ELISA for cell free nucleosome bound TTF-1 as described in EXAMPLE 1.

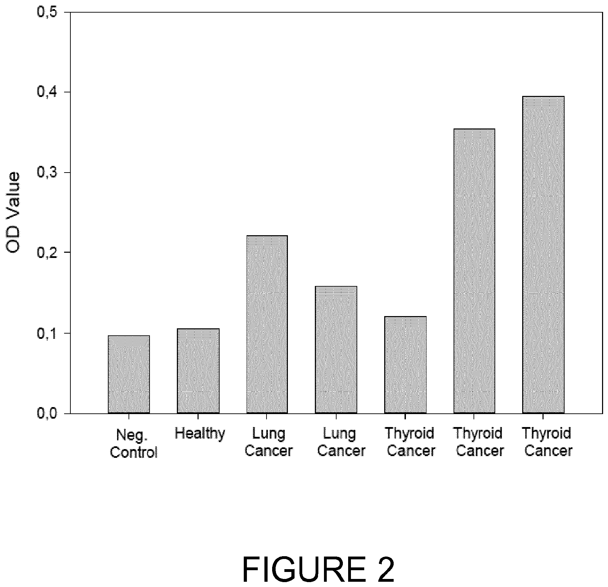

A higher ELISA signal, and a higher level of circulating cell free nucleosome-TTF-1 adduct, was observed for samples taken from thyroid cancer patients than from the healthy subject or the negative control. The level of circulating cell free nucleosome-TTF-1 adduct in the blood of lung cancer patients was slightly elevated. The results are shown in Figure 2.

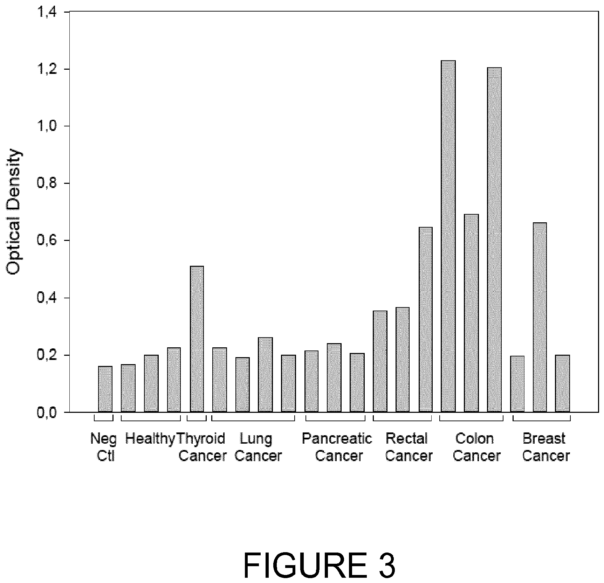

In a subsequent experiment, serum samples from 3 healthy subjects as well as samples from subjects diagnosed with thyroid, lung, pancreatic, rectal, colon and breast cancer were assayed for circulating cell free nucleosome-TTF-1 adduct level to ascertain the tissue specificity of the assay. The result for the single thyroid cancer patient assayed was strongly positive. Levels in all 4 lung cancer patients, all 3 pancreatic cancer patients and 2 of 3 breast cancer patients were low. Surprisingly; levels were elevated in all 6 colorectal cancer patients indicating that circulating cell free nucleosome-TTF-1 adduct levels may be used to identify cancers of unknown primary as colorectal primary cancers. This indicates that the tissue specificity of circulating cell free nucleosome-transcription factor adduct levels in the blood may not necessarily be the same as the specificity of transcription factor expression levels in cancer tissues removed at biopsy. The results are shown in Figure 3.

It will be clear to those skilled in the art that a nucleosome-TTF-1 adduct assay can be used to detect a cancer or to identify a cancer of unknown primary site of development where it is a thyroid or colorectal cancer and that the nucleosome-TTF-1 adduct can be used as a biomarker for this purpose.

### EXAMPLE 3

Serum samples were taken from 3 healthy subjects, 1 subject diagnosed with thyroid cancer, 3 subjects with lung cancer, 3 subjects with pancreatic cancer, 6 subjects with colorectal cancer (3 with colon cancer and 3 with rectal cancer) and 3 subjects with breast cancer. A commercially available 96 well research use only ELISA kit for CDX2 was purchased which included a solid phase antibody for CDX2 (caudal-type homeobox transcription factor-2) coated to the microtiter wells. CDX2 is a tissue specific transcription factor expressed in all or most biopsy material samples taken from colorectal cancers, but not in most samples from other cancers. The solid phase CDX2 antibody was used together with a biotinylated anti-nucleosome antibody, in a double antibody ELISA for cell free nucleosome bound CDX2 as described in EXAMPLE 1.

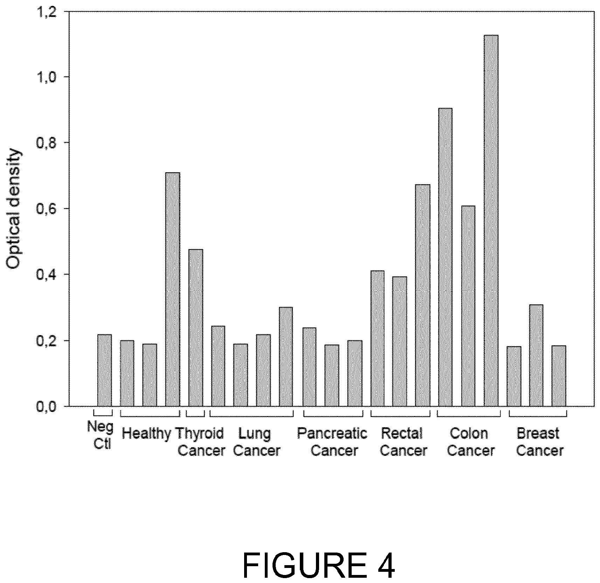

A higher ELISA signal, and a higher level of circulating cell free nucleosome-CDX2 adduct, was observed for samples taken from colorectal cancer patients than from healthy subjects or from subjects with other cancers or the negative control. One healthy subject and one subject with breast cancer also had elevated levels which may be false positive results. The results are shown in Figure 4 and indicate that circulating cell free nucleosome-CDX2 adduct levels can be used to detect a cancer or to identify a cancer of unknown primary site of development where it is a colorectal cancer and that the nucleosome-CDX2 adduct can be used as a biomarker for this purpose.

We successfully developed three prototype cell free nucleosome-tissue specific transcription factor adduct assays and all three exhibited clinical utility to identify the cancer of unknown primary site where a subject was already diagnosed with cancer. It is clear that this is a general effect and that similar nucleosome-tissue specific transcription factor assays may be developed for identification of a wide range of primary cancer diseases where cancer is diagnosed or detected but the site of development of the primary cancer is unknown. Similar tissue specific transcription factor expression is known to occur in cancer tissue samples removed by surgery or biopsy for many other cancers (Kandalaft and Gown, 2015). It will be clear to those skilled in the art that analogous blood or other body fluid based cell free nucleosome adduct assays similar to those described in EXAMPLES 1-3 for appropriate tissue specific chromatin fragments or tissue specific nucleosome-transcription factor or cofactor adducts can be used to detect cancer or to identify the cancer of unknown primary site for the wide range of cancers where such transcription factors have already been identified and/or any cancer where any such tissue specific transcription factor, tissue specific transcription cofactor or tissue specific combination of transcription factor and/or cofactor expression can be identified.

It will be understood that the embodiments described herein may be applied to all aspects of the invention.

### REFERENCES

Ballare et al., Nucleosome-driven transcription factor binding and gene regulation. Molecular Cell, 49(1): 67-79, 2013
Esteller, Cancer epigenomics: DNA methylomes and histone-modification maps Nature Reviews Genetics, 8: 286-298, 2007
Herranz and Esteller, DNA methylation and histone modifications in subjects with cancer: potential prognostic and therapeutic targets. Methods Mol Biol. 361: 25-62, 2007
Hervouet et al, Disruption of Dnmt1/PCNA/UHRF1 Interactions Promotes Tumorigenesis from Human and Mice Glial Cells PLoS ONE 5(6): e11333. doi:10.1371/journal.pone.0011333, 2010
Holdenrieder et al, Nucleosomes in serum of subjects with benign and malignant diseases. Int. J. Cancer (Pred. Oncol.), 95: 114-120, 2001
Holdenrieder et al, Cell-Free DNA in Serum and Plasma: Comparison of ELISA and Quantitative PCR. Clinical Chemistry, 51(8): 1544-1546, 2005
Holdenrieder and Stieber, Clinical use of circulating nucleosomes. Critical Reviews in Clinical Laboratory Sciences; 46(1): 1-24, 2009
Kandalaft and Gown, Practical Applications in Immunohistochemistry; Carcinomas of Unknown Primary Site. Arch Pathol. Lab. Med. doi: 10.5858/arpa.2015-0173-CP, 2015
Levy et al, Multiple Transcription Factor Elements Collaborate with Estrogen Receptor α to Activate an Inducible Estrogen Response Element in the NKG2E Gene. Endocrinology, 148(7): 3449-345, 2007
Lin et al., The expression levels of transcription factors T-bet, GATA-3, RORyt and FOXP3 in peripheral blood lymphocyte (PBL) of patients with liver cancer and their significance. Int. J. Med. Sci. 12(1): 7-16, 2015
Mariño-Ramírez et al, The Histone Database: an integrated resource for histones and histone fold-containing proteins. Database, doi: 10.1093/database/bar048, 2011
Muller et al, FHL2, a novel tissue-specific coactivator of the androgen receptor. The EMBO Journal 19(3): 359-369, 2000
Pihlajamaa et al, Tissue-specific pioneer factors associate with androgen receptor cistromes and transcription programs. The EMBO Journal 33(4): 312-326, 2014
Ricke and Bielinsky, Easy detection of chromatin binding proteins by the histone association. Assay Biol Proced Online, 7(1): 60-69, 2005
Salgame et al, An ELISA for detection of apoptosis. Nucleic Acids Research, 25(3): 680-681, 1997
Snyder et al., Cell-free DNA comprises an in vivo nucleosome footprint that informs its tissues-of origin. Cell, 164(1-2): 57-68, 2016
Takaku et al., GATA3-dependent cellular reprogramming requires activation-domain dependent recruitment of a chromatin remodeler. Genome Biology, 17(1), 2016
van Nieuwenhuijze et al, Time between onset of apoptosis and release of nucleosomes from apoptotic cells: putative implications for sysytemic lupus erythematosus. Ann. Rheum. Dis., 62: 10-14, 2003
Wittwer et al., Circulating nucleosomes and immunogenic cell death markers HMGB1, sRAGE and DNAse in patients with advanced pancreatic cancer undergoing chemotherapy. Int. J. Cancer, 133(11): 2619-2630, 2013
Yang and Schwartz, Epigenetic control of gene expression in the lung. Am. J. Respir. Crit. Care Med. 183(10): 1295-1301, 2011
Yoshida and Shimura, Isolation of nonhistone chromosomal protein from calf thymus. Biochimica et Biophysica Acta (BBA) - Protein Structure, 263(3): 690-695, 1972
Yu et al, Computational analysis of tissue-specific combinatorial gene regulation: predicting interaction between transcription factors in human tissues. Nucleic Acids Research 34(17): 4925-4936, 2006
Zhang and Glass, Towards an understanding of cell-specific functions of signal-dependent transcription factors. Journal of Molecular Endocrinology, 51: T37-T50, 2013
Zhao et al, Estrogen Signaling via Estrogen Receptor β. Journal of Biological Chemistry, 285(51): 39575-39579, 2010

## Claims

1. *In vitro* use of a tissue specific transcription factor-nucleosome adduct or transcription cofactor-nucleosome adduct as a biomarker in a biological fluid for the detection or diagnosis of a cancer in a subject, wherein the tissue specific transcription factor-nucleosome adduct or transcription cofactor-nucleosome adduct is used to identify the site of development of the cancer in the subject.

2. A method for determining the site of development of a cancer in a subject which comprises the steps of:
(i) contacting a biological fluid sample obtained from the subject with a first binding agent which binds to nucleosomes or a component thereof, or a tissue specific transcription factor or cofactor adducted to a nucleosome;
(ii) contacting the nucleosomes or sample with a second binding agent which binds to a tissue specific transcription factor or cofactor adducted to a nucleosome when the first binding agent binds to the nucleosome or a component thereof, or said second binding agent binds to a nucleosome or component thereof when the first binding agent binds to a tissue specific transcription factor or cofactor adducted to a nucleosome; and
(iii) detecting or quantifying the binding of said second binding agent to the adducted tissue specific transcription factor or cofactor, nucleosomes or a component thereof in the sample;
wherein the presence or level of the tissue specific transcription factor or cofactor adducted to a nucleosome is used as an indicator of the site of development of said cancer.

3. The method as defined in claim 2, which comprises the steps of:
(i) contacting a biological fluid sample obtained from the subject with a first binding agent which binds to nucleosomes or a component thereof;
(ii) contacting the nucleosomes or sample with a second binding agent which binds to a tissue specific transcription factor or cofactor adducted to a nucleosome; and
(iii) detecting or quantifying the binding of said second binding agent to the adducted tissue specific transcription factor or cofactor in the sample.

4. The method as defined in claim 2, which comprises the steps of:
(i) contacting a biological fluid sample obtained from the subject with a first binding agent which binds to a tissue specific transcription factor or cofactor;
(ii) contacting the sample with a second binding agent which binds to nucleosomes or a component thereof; and
(iii) detecting or quantifying the binding of said second binding agent to nucleosomes or a component thereof in the sample.

5. The *in vitro* use as defined in claim 1, or the method as defined in any one of claims 2 to 4, wherein the cancer is a primary cancer.

6. The *in vitro* use as defined in claim 1, or the method as defined in any one of claims 2 to 5, wherein the subject is a subject with metastatic cancer of unknown primary cancer.

7. The *in vitro* use as defined in claim 1, or the method as defined in any one of claims 2 to 6, wherein the subject has previously been diagnosed with cancer.

8. The *in vitro* use or method as defined in claim 7, wherein the subject was previously diagnosed with cancer using circulating tumour DNA (ctDNA) as a biomarker.

9. The method as defined in any one of claims 2 to 8, wherein the binding agent is an antibody, an antibody fragment or an aptamer.

10. The method as defined in any one of claims 2 to 9, wherein the binding agent which binds to nucleosomes or a component thereof, is directed to bind to a particular histone, histone modification, histone variant or isoform, or to DNA or a component thereof.

11. A method for assessment of an animal or a human subject for suitability for a medical treatment which comprises the steps of:
(i) detecting or measuring a tissue specific transcription factor or cofactor adducted to a nucleosome in a biological fluid of the subject as defined in the method of any one of claims 2 to 10; and
(ii) using the type of tissue specific transcription factor or cofactor adducted to a nucleosome detected to determine a suitable treatment for the subject.

12. A method for monitoring a treatment of an animal or a human subject which comprises the steps of:
(i) detecting or measuring a tissue specific transcription factor or cofactor adducted to a nucleosome in a biological fluid of the subject as defined in the method of any one of claims 2 to 10;
(ii) repeating the detection or measurement of the tissue specific transcription factor or cofactor adducted to a nucleosome in a biological fluid of the subject on one or more occasions; and
(iii) using any changes in the level of tissue specific transcription factor or cofactor adducted to a nucleosome detected as a parameter for any changes in the condition of the subject.

13. The *in vitro* use as defined in claim 1, or the method as defined in any one of claims 2 to 12 wherein the tissue specific transcription factor is selected from: GATA 3, CDX2, TTF-1, PAX8, WT1, NKX3.1, P63 or P40.

14. The *in vitro* use as defined in claim 1, or the method as defined in any one of claims 2 to 13, wherein the biological fluid is selected from: blood, serum or plasma.

## Patentansprüche

1. *In*-*vitro*-Verwendung eines gewebespezifischen Transkriptionsfaktor-Nukleosom-Addukts oder Transkriptions-Kofaktor-Nukleosom-Addukts als Biomarker in einer biologischen Flüssigkeit zum Erkennen oder zur Diagnose einer Krebserkrankung bei einem Patienten, wobei das gewebespezifische Transkriptionsfaktor-Nukleosom-Addukt oder Transkriptions-Kofaktor-Nukleosom-Addukt verwendet wird, um die Stelle einer Entwicklung des Krebses bei dem Patienten zu identifizieren.

2. Verfahren zum Bestimmen der Stelle einer Entwicklung eines Krebses bei einem Patienten, das die folgenden Schritte umfasst:
(i) Inkontaktbringen einer von dem Patienten erhaltenen Probe einer biologischen Flüssigkeit mit einem ersten Bindemittel, das sich an Nukleosomen oder eine Komponente davon bindet, oder mit einem gewebespezifischen Transkriptionsfaktor oder -kofaktor, der an einem Nukleosom angelagert ist;
(ii) Inkontaktbringen der Nukleosomen oder Probe mit einem zweiten Bindemittel, das sich an einen gewebespezifischen Transkriptionsfaktor oder -kofaktor bindet, der an einem Nukleosom angelagert ist, wenn sich das erste Bindemittel an das Nukleosom oder eine Komponente davon bindet, oder sich das zweite Bindemittel an ein Nukleosom oder eine Komponente davon bindet, wenn sich das erste Bindemittel an einen gewebespezifischen Transkriptionsfaktor oder - kofaktor bindet, der an einem Nukleosom angelagert ist; und
(iii) Erkennen oder Quantifizieren der Bindung des zweiten Bindemittels an dem angelagerten gewebespezifischen Transkriptionsfaktor oder -kofaktor, Nukleosomen oder einer Komponente davon in der Probe;
wobei das Vorhandensein oder Niveau des an einem Nukleosom angelagerten gewebespezifischen Transkriptionsfaktors oder -kofaktors als Indikator für die Stelle einer Entwicklung des Krebses verwendet wird.

3. Verfahren nach Anspruch 2, das die folgenden Schritte umfasst:
(i) Inkontaktbringen einer von dem Patienten erhaltenen Probe einer biologischen Flüssigkeit mit einem ersten Bindemittel, das sich an Nukleosomen oder eine Komponente davon bindet;
(ii) Inkontaktbringen der Nukleosomen oder Probe mit einem zweiten Bindemittel, das sich an einen gewebespezifischen Transkriptionsfaktor oder -kofaktor bindet, der an einem Nukleosom angelagert ist; und
(iii) Erkennen oder Quantifizieren der Bindung des zweiten Bindemittels an den angelagerten gewebespezifischen Transkriptionsfaktor oder -kofaktor in der Probe.

4. Verfahren nach Anspruch 2, das die folgenden Schritte umfasst:
(i) Inkontaktbringen einer von dem Patienten erhaltenen Probe einer biologischen Flüssigkeit mit einem ersten Bindemittel, das sich an einen gewebespezifischen Transkriptionsfaktor oder -kofaktor bindet;
(ii) Inkontaktbringen der Probe mit einem zweiten Bindemittel, das sich an Nukleosomen oder eine Komponente davon bindet; und
(iii)Erkennen oder Quantifizieren der Bindung des zweiten Bindemittels an Nukleosomen oder eine Komponente davon in der Probe.

5. *In*-*vitro*-Verwendung nach Anspruch 1 oder Verfahren nach in einem der Ansprüche 2 bis 4, wobei der Krebs ein Primärkrebs ist.

6. *In*-*vitro*-Verwendung nach Anspruch 1 oder Verfahren nach einem der Ansprüche 2 bis 5, wobei der Patient ein Patient mit metastasierendem Krebs von unbekanntem Primärkrebs ist.

7. *In*-*vitro*-Verwendung nach Anspruch 1 oder Verfahren nach einem der Ansprüche 2 bis 6, wobei bei dem Patienten vorangehend Krebs diagnostiziert wurde.

8. *In*-*vitro*-Verwendung oder Verfahren nach Anspruch 7, wobei bei dem Patienten vorangehend unter Verwendung von zirkulierender Tumor-DNA (ctDNA) als Biomarker Krebs diagnostiziert wurde.

9. Verfahren nach einem der Ansprüche 2 bis 8, wobei das Bindemittel ein Antikörper, ein Antikörperfragment oder ein Aptamer ist.

10. Verfahren nach einem der Ansprüche 2 bis 9, wobei das Bindemittel, das sich an Nukleosomen oder eine Komponente davon bindet, angeleitet wird, sich an ein bestimmtes Histon, eine bestimmte Histonmodifikation, eine bestimmte Histonvariante oder -isoform, oder an DNA oder eine Komponente davon zu binden.

11. Verfahren zur Beurteilung eines tierischen oder menschlichen Patienten auf Eignung für eine medizinische Behandlung, welche die folgenden Schritte umfasst:
(i) Erkennen oder Messen eines gewebespezifischen Transkriptionsfaktors oder -kofaktors, der an einem Nukleosom in einer biologischen Flüssigkeit des Patienten angelagert ist, wie in dem Verfahren nach einem der Ansprüche 2 bis 10 definiert; und
(ii) Verwenden der Art des gewebespezifischen Transkriptionsfaktors oder -kofaktors, der an einem erkannten Nukleosom angelagert ist, um eine geeignete Behandlung für den Patienten zu bestimmen.

12. Verfahren zum Überwachen einer Behandlung eines tierischen oder menschlichen Patienten, das die folgenden Schritte umfasst:
(i) Erkennen oder Messen eines gewebespezifischen Transkriptionsfaktors oder -kofaktors, der an einem Nukleosom in einer biologischen Flüssigkeit des Patienten angelagert ist, wie in dem Verfahren nach einem der Ansprüche 2 bis 10 definiert;
(ii) Wiederholen der Erkennung oder Messung des gewebespezifischen Transkriptionsfaktors oder -kofaktors, der an einem Nukleosom in einer biologischen Flüssigkeit des Patienten angelagert ist, bei einer oder mehreren Gelegenheiten; und
(iii)Verwenden von Änderungen des Niveaus des gewebespezifischen Transkriptionsfaktors oder -kofaktors, der an einem Nukleosom angelagert ist, das als Parameter für Änderungen des Zustands des Patienten erkannt wurde.

13. *In*-*vitro*-Verwendung nach Anspruch 1 oder Verfahren nach einem der Ansprüche 2 bis 12, wobei der gewebespezifische Transkriptionsfaktor aus Folgendem ausgewählt ist: GATA 3, CDX2, TTF-1, PAX8, WT1, NKX3.1, P63 oder P40.

14. *In*-*vitro*-Verwendung nach Anspruch 1 oder Verfahren nach einem der Ansprüche 2 bis 13, wobei die biologische Flüssigkeit aus Blut, Serum oder Plasma ausgewählt ist.

## Revendications

1. Utilisation *in vitro* d'un adduit de nucléosome-facteur de transcription spécifique à un tissu ou d'un adduit de nucléosome-cofacteur de transcription en tant que biomarqueur dans un fluide biologique pour la détection ou le diagnostic d'un cancer chez un sujet, dans laquelle l'adduit de nucléosome-facteur de transcription spécifique à un tissu ou l'adduit de nucléosome-cofacteur de transcription est utilisé pour identifier le site de développement du cancer chez le sujet.

2. Procédé de détermination du site de développement d'un cancer chez un sujet qui comprend les étapes :
(i) de mise en contact d'un échantillon de fluide biologique obtenu à partir du sujet avec un premier agent de liaison qui se lie à des nucléosomes ou à un composant de ceux-ci, ou un facteur ou cofacteur de transcription spécifique à un tissu ajouté à un nucléosome ;
(ii) de mise en contact des nucléosomes ou de l'échantillon avec un second agent de liaison qui se lie à un facteur ou cofacteur de transcription spécifique à un tissu ajouté à un nucléosome lorsque le premier agent de liaison se lie au nucléosome ou à un composant de celui-ci, ou ledit second agent de liaison se lie à un nucléosome ou à un composant de celui-ci lorsque le premier agent de liaison se lie à un facteur ou cofacteur de transcription spécifique à un tissu ajouté à un nucléosome ; et
(iii)de détection ou quantification de la liaison dudit second agent de liaison au facteur ou cofacteur de transcription spécifique à un tissu ajouté, à des nucléosomes ou à un composant de ceux-ci dans l'échantillon ;
dans lequel la présence ou le niveau du facteur ou cofacteur de transcription spécifique à un tissu ajouté à un nucléosome est utilisé comme indicateur du site de développement dudit cancer.

3. Procédé selon la revendication 2, qui comprend les étapes :
(i) de mise en contact d'un échantillon de fluide biologique obtenu à partir du sujet avec un premier agent de liaison qui se lie à des nucléosomes ou à un composant de ceux-ci ;
(ii) de mise en contact des nucléosomes ou de l'échantillon avec un second agent de liaison qui se lie à un facteur ou cofacteur de transcription spécifique à un tissu ajouté à un nucléosome ; et
(iii)de détection ou quantification de la liaison dudit second agent de liaison au facteur ou cofacteur de transcription spécifique à un tissu ajouté dans l'échantillon.

4. Procédé selon la revendication 2, qui comprend les étapes :
(i) de mise en contact d'un échantillon de fluide biologique obtenu à partir du sujet avec un premier agent de liaison qui se lie à un facteur ou cofacteur de transcription spécifique à un tissu ;
(ii) de mise en contact de l'échantillon avec un second agent de liaison qui se lie à des nucléosomes ou à un composant de ceux-ci ; et
(iii)de détection ou quantification de la liaison dudit second agent de liaison à des nucléosomes ou à un composant de ceux-ci dans l'échantillon.

5. Utilisation *in vitro* selon la revendication 1, ou procédé selon l'une quelconque des revendications 2 à 4, dans laquelle ou dans lequel le cancer est un cancer primaire.

6. Utilisation *in vitro* selon la revendication 1, ou procédé selon l'une quelconque des revendications 2 à 5, dans laquelle ou dans lequel le sujet est un sujet atteint d'un cancer métastatique d'un cancer primaire inconnu.

7. Utilisation *in vitro* selon la revendication 1, ou procédé selon l'une quelconque des revendications 2 à 6, dans laquelle ou dans lequel le sujet a été préalablement diagnostiqué avec un cancer.

8. Utilisation *in vitro* ou procédé selon la revendication 7, dans laquelle ou dans lequel le sujet a été préalablement diagnostiqué avec un cancer en utilisant l'ADN tumoral circulant (ADNc) en tant que biomarqueur.

9. Procédé selon l'une quelconque des revendications 2 à 8, dans lequel l'agent de liaison est un anticorps, un fragment d'anticorps ou un aptamère.

10. Procédé selon l'une quelconque des revendications 2 à 9, dans lequel l'agent de liaison qui se lie à des nucléosomes ou à un composant de ceux-ci, est dirigé pour se lier à une histone, une modification d'histone, un variant d'histone ou une isoforme particulier, ou à l'ADN ou à un composant de celui-ci.

11. Procédé d'évaluation de l'aptitude d'un animal ou d'un sujet humain à un traitement médical comprenant les étapes : (i) de détection ou mesure d'un facteur ou cofacteur de transcription spécifique à un tissu ajouté à un nucléosome dans un fluide biologique du sujet tel que défini dans le procédé selon l'une quelconque des revendications 2 à 10 ; et
(ii) d'utilisation du type de facteur ou cofacteur de transcription spécifique à un tissu ajouté à un nucléosome détecté pour déterminer un traitement approprié pour le sujet.

12. Procédé de surveillance d'un traitement d'un animal ou d'un sujet humain qui comprend les étapes :
(i) de détection ou mesure d'un facteur ou cofacteur de transcription spécifique à un tissu ajouté à un nucléosome dans un fluide biologique du sujet tel que défini dans le procédé selon l'une quelconque des revendications 2 à 10 ;
(ii) de répétition de la détection ou mesure du facteur ou cofacteur de transcription spécifique à un tissu ajouté à un nucléosome dans un fluide biologique du sujet à une ou plusieurs occasions ; et
(iii) d'utilisation de toute modification du niveau de facteur ou cofacteur de transcription spécifique à un tissu ajouté à un nucléosome détecté comme paramètre pour toute modification de l'état du sujet.

13. Utilisation *in vitro* selon la revendication 1, ou procédé selon l'une quelconque des revendications 2 à 12, dans laquelle ou dans lequel le facteur de transcription spécifique à un tissu est choisi parmi : GATA 3, CDX2, TTF-1, PAX8, WT1, NKX3.1, P63 ou P40.

14. Utilisation *in vitro* selon la revendication 1, ou procédé selon l'une quelconque des revendications 2 à 13, dans laquelle ou dans lequel le fluide biologique est choisi parmi : le sang, le sérum ou le plasma.
